# EUROPEAN PATENT APPLICATION

(11) **EP 4 261 245 A1**
(43) Date of publication of application: **18.10.2023**
(21) Application number: 21898305.4
(22) Date of filing: 15.09.2021
(51) Int. Cl.: C08J 3/24, C08J 3/075, C08J 3/12, C08L 5/08, C08K 5/1515, C08K 5/053, A61K 9/16, A61K 8/73, A61K 8/02, A61Q 19/00

(54) **METHOD FOR PRODUCING HIGHLY SWELLABLE HYALURONIC ACID BEAD GEL**

(30) Priority: 30.11.2020 KR 20200164683
(71) Applicant: Yu Co., Ltd., Ulsan 44496 (KR)
(72) Inventor: WOO, Sang Uk, Ulsan 44705 (KR); CHO, Kwang Yong, Ulsan 44715 (KR)
(74) Representative: Gulde & Partner
(86) International application number: PCT/KR2021/012624
(87) International publication number: WO 2022/114478

(57) **Abstract**

The present invention relates to a method for producing hyaluronic acid bead gels characterized by a high degree of swelling, the method comprising: (a) a reaction step of putting hyaluronic acid or a salt thereof in an aqueous alkali solution, adding a crosslinking agent, and then reacting the homogeneously mixed reactants; (b) a crosslinking reaction step of forming a crosslinked covalent bond by stirring the reaction mixture with oil; and (c) a step of purifying the product obtained in the crosslinking reaction step, wherein the hyaluronic acid bead gels are homogenously spherical. According to the method for producing hyaluronic acid bead gels of the present invention, transparent, uniform bead gels with a high degree of swelling can be produced. Such hyaluronic acid spherical bead gels exhibit sizes and decomposition time periods, viscoelasticity, and degrees of swelling that are selected for transparent bead forms, and are excellent in terms of biocompatibility and very stable to heat and enzymes.

## Description

### [Technical Field]

The present invention relates to a method for producing a homogenously spherical hyaluronic acid bead gel in which the hyaluronic acid is transparent and has a high degree of swelling by reperforming crosslinking reaction in a state of being dispersed in oil after crosslinking reaction.

The present invention can stably obtain a spherical hyaluronic acid bead that is transparent, has a high degree of swelling, a homogeneously spherical, and various sizes by controlling the ratio of oil and a hyaluronic acid solution without using emulsifiers, surfactants, solubilizers, and the like. In order to maintain a spherical shape in the obtaining step, a method of adding synthetic polymers such as PLGA and PCL, which are envelope forming materials, a method of producing an insoluble precipitate through ionic bonding, a method of precipitating in an organic solvent, or the like is not used. The spherical shape is maintained through two-step crosslinking in the hyaluronic acid solution dispersed in the oil phase without additives. In addition, it relates to a production method by applying a three-step purification method to produce a transparent bead gel by removing impurities such as oil and unreacted substances. The obtained spherical hyaluronic acid bead gel has a transparent spherical shape, shows the selected size, decomposition period, viscoelasticity, and degree of swelling, has excellent biocompatibility, and is very stable against heat and enzymes. The spherical hyaluronic acid bead gel having such characteristics according to the present invention can be used in a variety of ways, such as a filler for removing wrinkles, an implant, a drug delivery matrix, a cell carrier, a cell scaffold, an injection agent for joint, and cosmetics.

### [Background Art]

Hyaluronic acid ((HA), Hyaluronan, (C₁₄H₂₀NNaO₁₁)n (n>1000)) is a polymer existing in living organisms, and is a polysaccharide, called glycosaminoglycan. It has a structure which is composed of alternating D-glucuronic acid and N-acetylglucosamine, linked together via alternating β-1,3 and β-1,4 bonds. It is a water-soluble material with significantly high viscosity and elasticity, while its molecular weight ranges variably from 1,000 to 10,000,000Da (daltons) with its extensive structure of straight chain polysaccharide.

Hyaluronic acid has a strong moisturizing effect and has a strong lubricating function in a physical friction state. In addition, since it has very desirable advantages in efficacy and physical properties, such as a protective effect against bacterial invasion, it has a wide range of applications for cosmetic and medical purposes.

In order to develop hyaluronic acid, a biological tissue extraction method or a microorganism culturing method has been used basically. However, since a chicken comb extraction method causes many disadvantages such as virus invasion, impurities, and inflammatory reactions, a microorganism culturing production method that can control a molecular weight and productivity and can obtain high quality raw materials are becoming the mainstream.

In particular, it is a recent development trend that the use of hyaluronic acid is determined according to the range of a molecular weight of hyaluronic acid adjusted and produced by the microorganism culturing method. That is, an ultra-low molecular weight hyaluronic acid of 100,000 Da or less is mainly used for foods or cosmetics, while a low molecular weight hyaluronic acid with an average molecular weight of 1 million Da is utilized for developing an eye-drops raw material or its derivative. Hyaluronic acid with an average molecular weight of 3 million to 4 million Da is highly valuable when utilized as a raw material for a knee joint injection.

In addition, its utilization as an ophthalmic surgery adjuvant has been increasing. As an ultra-high molecular weight material in the body, it has been highlighted for the purpose of a raw material for anti-adhesive agent.

However, hyaluronic acid is easily decomposed in vivo or under conditions such as acids and alkalis, and has poor processability, so there is a limit to developing it for various uses. Accordingly, efforts are being made to develop substances with structurally stabilized hyaluronic acid. (Laurent, T.C. "The chemistry, biology and medical applications of hyaluronan and its derivatives" Portland Press Ltd, London, 1998).

Crosslinked hyaluronic acids are being developed for various uses, such as postoperative adhesion prevention films, gel-type adhesion prevention agents, wrinkle removal fillers, cosmetic aids, injection agents for j oint, drug delivery matrix and cell scaffolds. In particular, crossed-linked hyaluronic acids have been actively studied for commercial use such as wrinkle removal fillers and cosmetic aids. (F. Manna, M. Dentini, P. Desider, O. De Pita, E. Mortilla, B. Maras, Journal of European Academy of Dermatology and Venereology, 13 (1999) 183-192).

A method of mixing and using a chemical method and a physical method is a generally well-known method for producing beads composed of crosslinked hyaluronic acid. As known methods for separating the beads, there are precipitation through a fine nozzle, lipidation and encapsulation of the outside, dispersing the solubilized biodegradable polymer using a surfactant and obtaining beads by solvent extraction and evaporation, and the like.

In the physical method, the high uniformity of the particles is affected by the shape of the impeller and the stirring speed, and a high stirring speed must be maintained in most cases. In the method using a fine nozzle, an appearance similar to that of a pomegranate fruit is generated so it does not maintain a perfect spherical shape. In the case that spherical beads are obtained by lipidation and encapsulation on the outside of the bead, the beads are broken by a physical external force and the liquid phase inside flows out. When using the solvent extraction evaporation method, surfactants and organic solvents harmful to the human body are necessarily used, and it is necessary to completely remove them from the final product.

As other general methods for producing beads composed of crosslinked hyaluronic acid, there is a method of using an excessive amount of a substance representing a cation to form an insoluble precipitate through ionic bonding with anionic hyaluronic acid, a method of using an excessive amount of a crosslinking agent to form an insoluble form, and then drying for physically granulation, a method of binding a hydrophobic substance to the carboxyl group of hyaluronic acid, dissolving it in an organic solvent, forming microbeads using an emulsifier in two different phase-separating solvents, and collecting the microbeads by solvent extraction. For example, U.S. Patent Nos. 6,066,340 and 6,039,970 disclose the use of hyaluronic acid derivatives synthesized by combining ethyl alcohol or benzyl alcohol with carboxyl groups of hyaluronic acid. This substance is insoluble in water but soluble in organic solvents such as dimethyl sulfoxide. There are examples of producing solid microbeads by emulsion solvent extraction using such hydrophobic hyaluronic acid derivatives.

In addition, research is being conducted to prepare beads with chitosan and use them as a drug delivery matrix. (S. T. Lim, G. P. Martin, D. J. Berry and M. B. Brown, Journal of Controlled Release, 2-3, 66(2000), 281-292, S. T. Lim, B. Forbes, D. J. Berry, G. P. Martin and M. B. Brown, International Journal of Pharmaceutics, 1, 231(2002), 73-82).

In addition, in literature related to the production of hyaluronic acid beads crosslinked by 1,3-butadiene diepoxide, conditions for performing a crosslinking reaction by adding 1,3-butandiene diepoxide after stirring a mixture of hyaluronic acid and vegetable oil in an alkaline aqueous solution are disclosed. In this condition, by applying an excess of 0.4 mL of 3-butandiene diepoxide (approximately 800 mol % compared to the crosslinking reaction site) to 100 mg of hyaluronic acid, the swelling (%) is around 500 %, which is too hard and has an uneven shape. In addition, due to the use of an excessive amount of crosslinking agent, it has limitations in application fields. (Polymer(Korea), Vol.29, No.5, pp445-450, 2005).

In addition, in the method for producing hyaluronic acid microbeads, hyaluronic acid microbeads are produced by dropping droplets of an aqueous solution of hyaluronic acid through a nozzle under the pressure of compressed air through a microbead generating device into the crosslinking solution being stirred. In this method, there are problems in that safety issues may occur due to the use of divinyl sulfone as a crosslinking agent, the shape between particles are uneven and uniformity is poor, and expensive equipment must be used. (Biomaterials Research (2009) 13(3) : 105-108, Biomaterials Research (2010) 14(4) : 157-160, Chun et al, Biomaterials Research (2016) 20:24, Microbeads using polysaccharides and preparation method thereof(KR Patent Registered No. 1005489650000(2006.01.26))).

In addition, there is a method of producing solid bead-type emulsion beads by dropping in a freezing refrigerant of -10 °C to - 200 °C, but there are many components required and there is no purification process. Accordingly, There is a limit to its application field use. (Preparation of emulsion having a round bead form by freezing at low temperature and thus produced emulsion having a round bead form, KR Patent Registered No. 1015532330000(2015.09.09)).

In addition, the literature related to the production of hyaluronic acid beads crosslinked by BDDE discloses a manufacturing method including dissolving hyaluronic acid in an alkaline aqueous solution overnight at 10 °C to lower the molecular weight, adding excess BDDE (20 mg of BDDE per 100 mg of hyaluronic acid, about 40 mol % compared to weak crosslinking reaction site), stirring reaction with olive oil, collecting through a filer, and purifying with 70 % acetone aqueous solution, distilled water (DW), and PBS. Here, due to the high chemical strain close to 20 to 30 % and the low degree of swelling of 18.99 to 23.02, it has a too hard and non-uniform shape, and its use in the application field is limited. (ACS Omega 2019, 4, 9, 13834-13844, Hyaluronic Acid-Based Hybrid Hydrogel Microspheres with Enhanced Structural Stability and High Injectability).

In addition, there is a method for producing nano/microbeads by crosslinking hyaluronic acid with ligands selected from the group consisting of alendronate (ALD), adenosine diphosphate (ADP), and adenosine triphosphate (ATP) and dissolving the crosslinked hyaluronic acids, and dropping the dissolved hyaluronic acids on multivalent cations. However, due to problems with safety and stability, its use in the application field is limited. (a method for producing hyaluronic acid microbeads and a use of the hyaluronic acid microbeads, KR Patent Registered No. 1021085520000(2020.04.29)).

However, as hyaluronic acid is a substance with very high hydrophilicity, there have been limitations in producing homogeneously spherical beads that are transparent and have a high degree of swelling by the above generally known methods. Hyaluronic acid having an aqueous phase has a very high hydrophilicity in an oil phase, so it is difficult to remove moisture by solvent evaporation, and when an organic solvent is added by solvent extraction, the particles are entangled with each other, so that beads of uniform size was not produced. In addition, in the method for producing hyaluronic acid beads known to date, an insoluble precipitate is produced by using an excessive amount of a substance representing a cation through an ionic bond with anionic hyaluronic acid, or an insoluble form is formed by using an excessive amount of a crosslinking agent and then dried to obtain physical granulation, or a hydrophobic substance is bound to the carboxyl group of hyaluronic acid and dissolved in an organic solvent and solvent extraction is performed. However, the beads produced in this way have a fairly small particle size, non-uniform shape, hard opaque particles with a very low degree of swelling in an aqueous solution, and thus, there are limitation in their applications.

As described above, due to the unique material properties of hyaluronic acid, conventional techniques could not be applied to a process for producing beads with a very high degree of swelling in an aqueous solution while maintaining a homogeneously spherical shape and transparency.

### [DISCLOSURE]

### [Technical Problem]

In order to solve the above technical problem, an object of the present invention is to provide a homogenously spherical hyaluronic acid bead gel with transparency and a high degree of swelling.

That is, an object of the present invention is to provide a homogenously spherical hyaluronic acid bead gel with transparency and a high degree of swelling that can be usefully used in medical devices, pharmaceuticals, cosmetics, and food industries due to its excellent biocompatibility in various environments inside and outside the body by controlling homogenously spherical shape and size, deposition period of spherical hyaluronic acid bead gel through crosslinking, and by having a high degree of swelling in an aqueous solution, and an excellent physical stability.

Another object of the present invention is to provide a method for producing the spherical hyaluronic acid bead gel through a highly efficient two-step reaction process using a small amount of crosslinking agent forming a spherical shape and a three-step purification process. This method does not perform the conventional methods such as a method of lipidation and encapsulation of the outside of spherical beads, a method of using emulsifiers and surfactants, a method of forming an insoluble form with an excessive amount of crosslinking agent and then drying and physical granulating, a method of combining hydrophobic substances and dissolving them in an organic solvent and then phase separation are not performed. Thus, it is suitable for providing a transparent spherical hyaluronic acid bead gel with high-purity.

After extensive research and numerous experiments to achieve this object, the present inventors have produced the hyaluronic acid gel by controlling the size of beads through the relative ratio of a hyaluronic acid solution and oil without using chemical emulsifiers and chemical surfactants, and by a high-efficiency chemical bond by a two-step reaction process using a small amount of crosslinking agent and a three-step purification process. The spherical hyaluronic acid bead gel not only has a high-purity, homogenous and selectively sized transparent spherical shape, but also has a characteristic that the shape does not decompose even under 121 °C sterilization conditions, and excellent biocompatibility, viscoelasticity, swelling, stability, etc., so that the invention has been completed.

### [Technical Solution]

In order to solve the above technical problems, the present invention provides a method for producing a hyaluronic acid bead gel with a high degree of swelling, comprising (a) a reaction step of adding hyaluronic acid or a salt thereof in an alkali aqueous solution , adding a crosslinking agent, and then reacting a homogeneously mixed reactant; (b) a crosslinking reaction step of forming a crosslinked covalent bond by stirring the reactant with oil; and (c) a step of purifying a product obtained in the crosslinking reaction step, wherein the hyaluronic acid bead gel is homogenously spherical.

In another embodiment of the present invention, a degree (%) of swelling of the hyaluronic acid bead gel finally obtained through the purification process may be 3,000 % or more.

As another embodiment of the present invention, the production method of the present invention is characterized by not using an emulsifier, a chemical surfactant and a chemical solubilizer.

As another embodiment of the present invention, the production method of the present invention is characterized in that in the step (a), the crosslinking agent is added in a range of 1 to 30 equivalent % based on a repeating unit of the hyaluronic acid or salt thereof.

The crosslinking agent is easily decomposed and cannot maintain the shape if the crosslinking agent is added in less than 1 equivalent % based on the repeating unit of hyaluronic acid or its salt. Also, If the crosslinking agent is added in excess of 30 equivalent %, the frequency of side effects due to the use of a high amount of the crosslinking agent increases, and it is also undesirable in terms of ease of use due to hard physical properties with a low degree of swelling.

As another embodiment of the present invention, in the step (a), a concentration of the hyaluronic acid or salt thereof in the reactant is 1 to 30 % by weight, preferably 5 to 20 % by weight, based on 0.1 to 10 N alkali aqueous solution .

If the concentration of hyaluronic acid or its salt is less than 1% by weight based on the alkali aqueous solution of 0.1 to 10 N, crosslinking is not properly performed and it is difficult to maintain the shape, which is not preferable. In addition, when this concentration exceeds 30% by weight, homogeneous mixing is difficult due to too high concentration, resulting in non-uniform reaction, and too high crosslinking causes hard properties, which is not preferable.

In another embodiment of the present invention, the step (a) may be performed at a reaction temperature of 5 to 50 °C and a reaction time of 30 minutes to 24 hours.

In another embodiment of the present invention, the step (b) may be performed at a reaction temperature of 5 to 50 °C and a reaction time of 1 hour to 96 hours. It is preferable to perform the reaction at a stirring speed of 100 rpm to 2,000 rpm.

In another embodiment of the present invention, in the step (b), a weight ratio of the hyaluronic acid or its salt alkali aqueous solution to which the crosslinking agent is added and the oil may be 1:1 to 1:9.

After crosslinking and covalent bonding in the step (c), precipitation is performed using various types of organic solvents to remove moisture remaining in the crosslinked product, and the crosslinked hyaluronic acid beads are collected while washing several times to remove oil..

In another embodiment of the present invention, the crosslinking agent may be one or more selected from the group consisting of 1,4-butandiol diglycidyl ether(BDDE), ethylene glycol diglycidyl ether(EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, and sorbitol polyglycidyl ether.

In another embodiment of the present invention, the oil may be one or more selected from the group consisting of mineral oil, medium chain triglycerides, menhaden oil, linseed oil, coconut oil, cod oil (fish oil), soya bean oil, whale oil, cotton seed oil, sesame seed oil, peanut oil, olive oil, lard oil, palms oil, corn oil, rapeseed oil, bone oil, china wood oil, and castor oil.

In another embodiment of the present invention, the purification process preferably includes (a) step of collecting the hyaluronic acid bead gel that has undergone the crosslinking reaction step; (b) a first purification step of washing the collected spherical gel with one or more selected from distilled water, an acidic solution, an aqueous solution containing an acid salt and PBS; (c) a second purification step of washing the hyaluronic acid bead gel that has undergone the first purification step with an organic solvent aqueous solution; (d) a third purification step of washing the hyaluronic acid bead gel that has undergone the second purification step with one or more selected from distilled water, an acidic solution, an aqueous solution containing an acid salt, and PBS; and (e) separating and collecting the hyaluronic acid bead gel obtained in the third purification step.

The hyaluronic acid or its crosslinked salt that has crosslinked to a certain level in the purification step is washed several times with distilled water, an acidic solution or an aqueous solution containing a salt and PBS in the step (b) to remove unreacted crosslinking agent and hydroxide ions.

The purified bead gel in the step (b) is precipitated in an organic solvent again, and then swollen in an organic solvent aqueous solution for a certain period of time in the step (c), and then washed several times with an organic solvent to remove the oil and an unreacted crosslinking agent and impurities;

In the step (d), the unreacted crosslinking agent, impurities, and organic solvent are removed by washing again several times with distilled water, an acidic solution or an aqueous solution containing salt, and PBS, and finally, in the step (e), the bead gel is swollen to produce the hyaluronic acid bead gel.

In another embodiment of the present invention, the organic solvent may use one or more selected from DMSO, DMF, acetonitrile, methanol, THF, acetone, an acetone aqueous solution, and alcohol having 2 to 6 carbon atoms.

In another embodiment of the present invention, the organic solvent aqueous solution used in the swelling step preferably has a weight ratio of distilled water to an organic solvent of 9:1 to 1:9.

### [Advantageous Effect]

As described above, in the method for producing a spherical hyaluronic acid bead gel of the present invention, an emulsifier is not used in an oil phase, and the type and relative ratio of various types of oils and aqueous solutions are controlled to control the size and physical properties of the spherical hyaluronic acid bead gel.

In addition, the present invention can obtain high crosslinking efficiency and yield while using a small amount of crosslinking agent through a two-step crosslinking reaction, and also can control the transparency and physical properties of spherical hyaluronic acid bead gel through a three-step purification method.

The hyaluronic acid spherical bead gel produced through the present invention has a transparent spherical shape, exhibits a selected size, decomposition period, viscoelasticity, and degree of swelling, has excellent biocompatibility, and is very stable against heat and enzymes.

### [Description of Drawings]

FIG. 1 is a diagram of observing with an optical microscope that transparent spherical hyaluronic acid bead gels are swollen in PBS.
FIG. 2 is a diagram of observing with an optical microscope that amorphous hyaluronic acid bead gels produced in Comparative Example 5 are swollen in PBS.
FIG. 3 is a diagram showing the results of particle size analysis of the spherical hyaluronic acid bead gel produced in Example 1.
FIG. 4 is a diagram showing the results of particle size analysis of the spherical hyaluronic acid bead gel produced in Example 2.
FIG. 5 is a diagram showing the results of particle size analysis of the spherical hyaluronic acid bead gel produced in Example 3.
FIG. 6 is a diagram showing the results of rheological analysis of the spherical hyaluronic acid bead gel produced in Examples 1, 2, and 3.
FIG. 7 is a diagram showing the results of measuring the extrusion force of the spherical hyaluronic acid bead gel produced in Examples 1, 2, and 3.
FIG. 8 is a diagram of observing the properties of the spherical hyaluronic acid bead gel produced in Examples 1, 2, and 3 after passing through an injection needle.

### [Best Mode for Invention]

In the present invention, when producing a spherical hyaluronic acid bead gel, an emulsifier is not used in an oil phase, and the bead size is adjusted by the type and relative ratio of the hyaluronic acid solution and oil, and thus, a spherical hyaluronic acid bead gel that is chemically coupled with a small amount of a crosslinking agent (polyfunctional epoxide) having two or more epoxy reactive groups and a production method thereof are provided.

### [Mode for Invention]

The size of the spherical hyaluronic acid bead gel is not particularly limited, but the average diameter of the microparticles is preferably 50 to 2,000 um. Depending on the size of the spherical hyaluronic acid bead gel, its medial application range can be diversified.

In the present invention, the term hyaluronic acid briefly refers to a form of an acid and its salt. The hyaluronic acid salt includes both inorganic salts such as sodium hyaluronate, potassium hyaluronate, calcium hyaluronate, magnesium hyaluronate, zinc hyaluronate, cobalt hyaluronate and organic salts such as tetrabutylammonium hyaluronate.

In order to form a spherical hyaluronic acid with transparency, high swelling, and uniformity and to obtain various physical properties and biocompatibility, the molecular weight of the hyaluronic acid or its salt is not particularly limited and is preferably 100,000 to 5,000,000 daltons.

The production method according to the present invention includes a first reaction step of adding hyaluronic acid or a salt thereof in an alkali aqueous solution , adding a small amount of crosslinking agent, and homogeneously mixing and reacting the mixture, a second crosslinking reaction step of dispersing the reaction mixture in various types of oils, a first purification step of collecting the reactant that has undergone this reaction and purifying the reactant with distilled water, an acidic solution or an aqueous solution containing a salt and PBS to remove unreacted materials and impurities, a second purification step of purifying with an organic solvent aqueous solution, and a third purification step of purifying with distilled water, an acidic solution or an aqueous solution containing a salt, and PBS.

Other ingredients may be further included in the spherical hyaluronic acid bead gel of the present invention within a range that does not impair the effect of the present invention.

As described above, since hyaluronic acid used herein is a concept that includes both an acid form and a salt thereof, in the production method of the present invention, "hyaluronic acid aqueous solution" refers to all of an aqueous solution of hyaluronic acid, an aqueous solution of a salt of hyaluronic acid, and a mixed aqueous solution of hyaluronic acid and hyaluronic acid salt.

The alkali reagent used for producing the alkali aqueous solution dissolving the hyaluronic acid is not particularly limited, but preferably, reagents such as sodium hydroxide, potassium hydroxide, and ammonia hydroxide are used. The concentration of the alkali aqueous solution is not particularly limited, and the concentration is preferably 0.1 to 10 N for dissolution of hyaluronic acid and crosslinking reaction.

The crosslinking agent reacts with an alcohol group of hyaluronic acid or a salt thereof to form an ether bond to crosslink hyaluronic acid or a salt thereof. It is known that the reaction between an epoxy functional group and an alcohol is performed at a high temperature and in an alkaline condition, and as the reaction proceeds, no by-products are generated because it is attached to hyaluronic acid or a salt thereof. However, in the case that the reaction efficiency is low, unreacted epoxy functional groups among several epoxy functional groups of the crosslinking agent combined with hyaluronic acid or salts thereof remain in hyaluronic acid or salts thereof, and thus, there is a risk that these remaining unreacted epoxy functional groups will be used with substances in vivo later. Thus, additional reactions and processes are required to minimize this risk or convert the epoxy functional group into an inactive functional group by hydrolysis. In addition, in the case that an excessive amount of crosslinking agent is added, the risk of unreacted crosslinking agent remaining in the crosslinking substance of hyaluronic acid or its salt is very high. Therefore, in order to minimize this risk, inducing a high-efficiency reaction using an appropriate amount of crosslinking agent as described in the present invention is highly desirable not only in terms of biocompatibility of the spherical hyaluronic acid bead gel but also in terms of production process efficiency.

Hyaluronic acid or its salt-alkali aqueous solution homogeneously mixed with the crosslinking agent is mixed with various types of oil, and then appropriately stirred and crosslinked at a certain temperature. Hyaluronic acid or its salt-alkali aqueous solution dispersed in the oil phase gradually changes to a high concentration as the solvent evaporates, thereby increasing the actual reaction concentration and maximizing the efficiency of the reaction. Accordingly, a spherical hyaluronic acid bead gel with excellent physical properties can be produced using a small amount of crosslinking agent. Therefore, in the hyaluronic acid crosslinking reaction process described above, as the crosslinking agent is mixed at a low concentration and the solvent evaporates, the crosslinking reaction is performed at a relatively high concentration. Therefore, the above process can simultaneously take advantage of both the advantages of using a low concentration of crosslinking agent and the advantages of using a high concentration of crosslinking agent. This spherical hyaluronic acid bead gel production method can produce a spherical hyaluronic acid bead gel having strong and special physical properties, as well as having the efficiency of the crosslinking reaction at the same time.

In addition, when the hyaluronic acid or its salt-alkali aqueous solution containing the crosslinking agent is mixed with various types of oil, the physical properties of the spherical hyaluronic acid bead gel may be controlled by controlling the mixing time and the oil ratio to the aqueous solution.

In the absence of an emulsifier, the size of the spherical hyaluronic acid bead gel is controlled by the stirring speed, the type of oil, and the ratio of oil to an aqueous solution. The higher the viscosity of the oil phase, the smaller the size of the spherical hyaluronic acid spherical bead gel. This seems to be because hyaluronic acid and its salt-alkali aqueous solution, which have a relatively higher viscosity than that of the oil phase, are more smoothly dispersed as the difference in viscosity between the two phases decreases.

In addition, the size of the beads decreases as the stirring speed increases and the viscosity of the aqueous solution of hyaluronic acid and its salt alkali decreases.

After the covalent crosslinking, an organic solvent containing DMSO, DMF, acetonitrile, methanol, THF, acetone, acetone aqueous solution, alcohol having 2 to 6 carbon atoms such as ethanol, or alcohol aqueous solution may be used in collecting the bead gel.

Through several experiments, the present inventors have observed that the physical properties of the produced spherical hyaluronic acid bead gel are changed according to the characteristics of mixing the alkali aqueous solution of hyaluronic acid or its salt with the various organic solvents and oil phases described above, and have found that as the polarity index of the organic solvent is closer to distilled water, the transparency of the produced spherical hyaluronic acid bead gel increases and the complex viscosity decreases, so that the inventors proposed a method for producing a spherical hyaluronic acid bead gel with various physical properties.

The temperature during the crosslinking reaction of the mixture is not particularly limited, but is preferably 5 °C to 50 °C, more preferably 15 °C to 40 °C. In addition, the duration for forming the crosslinks in the reaction solution is not particularly limited, but the longer the reaction time, the lower the physical viscoelasticity of the spherical hyaluronic acid bead gel produced by hydrolysis of hyaluronic acid under alkaline conditions. Thus, the reaction time is preferably 30 minutes to 24 hours in the first step and 1 hour to 96 hours in the second step.

After the crosslinking reaction of the hyaluronic acid or its salt, an organic solvent is used to remove oil from the spherical hyaluronic acid bead gel product. Diethyl ether, DMSO, DMF, acetonitrile, methanol, THF, acetone, an aqueous acetone solution, an alcohol having 2 to 6 carbon atoms such as ethanol, or alcohol aqueous solution may be used as the organic solvent.

The oil-removed spherical hyaluronic acid bead gel product is washed several times in distilled water, an acidic solution, or an aqueous solution containing salt and PBS to remove unreacted crosslinking agent and hydroxide ions.

The washing efficiency of unreacted substances can be increased by using the aqueous solution to which the salt is added. In this case, an sodium chloride aqueous solution, a phosphoric acid aqueous solution, and a sodium chloride aqueous solution containing a phosphate are most suitable.

The purified spherical hyaluronic acid bead gel can be swollen in PBS and sterilized at 121 °C to finally produce a spherical hyaluronic acid bead gel that can be applied to a living body. The spherical hyaluronic acid bead gel is collected in a precipitated state using an organic solvent or an organic solvent aqueous solution, dried under nitrogen, air, heat, and reduced pressure, and the spherical hyaluronic acid bead gel is classified by size and type according to the purpose of medical application, and re-swelling in PBS and sterilized at 121 °C, so that a spherical hyaluronic acid bead gel that can be applied to a living body can be finally produced.

As the organic solvent used for precipitating the spherical hyaluronic acid bead gel, DMSO, DMF, acetonitrile, methanol, THF, acetone, acetone aqueous solution, an alcohol having 2 to 6 carbon atoms such as ethanol, or alcohol aqueous solution may be used.

The hyaluronic acid or crosslinked salt thereof produced by the present invention may be variously separated and/or purified by conventional methods known in the art, and typical examples thereof include distillation (including distillation under atmospheric pressure and vacuum distillation), recrystallization, column chromatography, ion exchange chromatography, gel chromatography, affinity chromatography, thin layer chromatography, phase separation, solvent extraction, dialysis, and washing. The separation and/or purification may be performed after each process or after a series of reactions when producing hyaluronic acid derivative microbeads.

The resulting spherical hyaluronic acid bead gel according to the present invention can be sterilized at 121 °C and has no side effects when introduced into the body, and can provide properties that can be used as a wrinkle removal filler, a cosmetic aid, an injection agent for joint, and an anti-adhesion agent. Also, it can be used as a vehicle for delivering a specific drug or cell to a specific living body site or as a cell scaffold for tissue engineering, as a drug delivery matrix or cell delivery matrix.

Hereinafter, the present invention will be described in detail by examples.

However, the following examples are only to illustrate the present invention, and the content of the present invention is not limited by the following examples.

### Examples

### [Example 1] Production of spherical hyaluronic acid bead gels

Sodium hyaluronate (molecular weight: 1,000,000) at a concentration of 50 mg/ml was added to 0.25 N 200 mL NaOH solution, and 0.4032 g of butanediol diglycidyl ether (BDDE) was added at the same time (Equivalent ratio of BDDE to hyaluronic acid repeating unit: 8 mol%). After mixing them for 30 minutes using a self-rotating centrifugal mixer, a first-step reaction was performed at 40 °C for 1 hour. The above reactant was dropped onto 600 g of MCT oil phase. While stirring the reactant at a speed of 700 rpm, a second-step reaction was performed at 40 °C for 24 hours.

After 24 hours, 800 ml of isopropanol was slowly added to collect the hyaluronic acid beads dispersed in the mixed solution. Washing was performed 3 times with isopropanol. The collected hyaluronic acid beads were washed 5 times with 5L PBS each. Precipitation was performed by adding 1 L of isopropanol to the washed hyaluronic acid beads. It was repeated three times and isopropanol was exchanged. The precipitate was swollen for 1 hour by adding 1.5 L of 30% isopropanol. After swelling, it was repeated three times and isopropanol was changed. The precipitate was again washed 5 times with 5 L PBS each.

Some of the washed spherical hyaluronic acid bead gels were swollen in PBS and sterilized at 121 °C to produce a spherical hyaluronic acid bead gel, and some of the other spherical hyaluronic acid bead gels were produced as spherical hyaluronic acid bead gels of a size and shape suitable for each application after going through an organic solvent precipitation process, a nitrogen gas drying process, a classification process, a PBS re-swelling process, and a 121 °C sterilization process.

### [Example 2] Production of spherical hyaluronic acid bead gels

Sodium hyaluronate (molecular weight: 1,000,000) at a concentration of 50 mg/ml was added to 0.25 N 200 mL NaOH solution, and 0.4536 g of butanediol diglycidyl ether (BDDE) was added at the same time (Equivalent ratio of BDDE to hyaluronic acid repeating unit: 9 mol%). After mixing them for 30 minutes using a self-rotating centrifugal mixer, a first-step reaction was performed at 40 °C for 1 hour. The above reactant was dropped onto 600 g of MCT oil phase. While stirring the reactant at a speed of 700 rpm, a second-step reaction was performed at 40 °C for 24 hours.

After 24 hours, 800 ml of isopropanol was slowly added to collect the hyaluronic acid beads dispersed in the mixed solution. Washing was performed 3 times with isopropanol. The collected hyaluronic acid beads were washed 5 times with 5L PBS each. Precipitation was performed by adding 1 L of isopropanol to the washed hyaluronic acid beads. It was repeated three times and isopropanol was exchanged. The precipitate was swollen for 1 hour by adding 1.5 L of 30% isopropanol. After swelling, it was repeated three times and isopropanol was changed. The precipitate was again washed 5 times with 5 L PBS each.

Some of the washed spherical hyaluronic acid bead gels were swollen in PBS and sterilized at 121 °C to produce a spherical hyaluronic acid bead gel, and some of the other spherical hyaluronic acid bead gels were produced as spherical hyaluronic acid bead gels of a size and shape suitable for each application after going through an organic solvent precipitation process, a nitrogen gas drying process, a classification process, a PBS re-swelling process, and a 121 °C sterilization process.

### [Example 3] Production of spherical hyaluronic acid bead gels

Sodium hyaluronate (molecular weight: 1,000,000) at a concentration of 50 mg/ml was added to 0.25 N 166 mL NaOH solution, and 0.4183 g of butanediol diglycidyl ether (BDDE) was added at the same time (Equivalent ratio of BDDE to hyaluronic acid repeating unit: 10 mol%). After mixing them for 30 minutes using a self-rotating centrifugal mixer, a first-step reaction was performed at 40 °C for 1 hour. The above reactant was dropped onto 332 g of MCT oil phase. While stirring the reactant at a speed of 700 rpm, a second-step reaction was performed at 40 °C for 24 hours.

After 24 hours, 500 ml of isopropanol was slowly added to collect the hyaluronic acid beads dispersed in the mixed solution. Washing was performed 3 times with isopropanol. The collected hyaluronic acid beads were washed 5 times with 5L PBS each. Precipitation was performed by adding 1 L of isopropanol to the washed hyaluronic acid beads. It was repeated three times and isopropanol was exchanged. The precipitate was swollen for 1 hour by adding 1.5 L of 30% isopropanol. After swelling, it was repeated three times and isopropanol was changed. The precipitate was again washed 5 times with 5 L PBS each.

Some of the washed spherical hyaluronic acid bead gels were swollen in PBS and sterilized at 121 °C to produce a spherical hyaluronic acid bead gel, and some of the other spherical hyaluronic acid bead gels were produced as spherical hyaluronic acid bead gels of a size and shape suitable for each application after going through an organic solvent precipitation process, a nitrogen gas drying process, a classification process, a PBS re-swelling process, and a 121 °C sterilization process.

### Industrial applicability

### [Comparative Example 1] Production of hyaluronic acid bead gels

Sodium hyaluronate (molecular weight: 1,000,000) at a concentration of 100 mg/ml was produced in 0.25 N 100 mL NaOH solution. The reactant was dropped onto 500 g of MCT oil phase. The reactant was stirred at a speed of 700 rpm. 44 g of butanediol diglycidyl ether (BDDE) as a crosslinking agent was added. The reaction was performed at 27 °C for 24 hours.

After 24 hours, 500 ml of isopropanol was slowly added to collect the hyaluronic acid beads dispersed in the mixed solution. Washing was performed 3 times with isopropanol. The collected hyaluronic acid beads were washed 2 times with 5L distilled water each. Precipitation was performed by adding 1 L of isopropanol to the washed hyaluronic acid beads. It was repeated three times and isopropanol was exchanged. The precipitate was again washed 2 times with 5 L distilled water each.

The washed hyaluronic acid bead gels were swollen in PBS and sterilized at 121 °C to produce a hyaluronic acid bead gel.

### [Comparative Example 2] Production of hyaluronic acid bead gels

A hyaluronic acid bead gel was produced in the same manner as in Comparative Example 1, except that 49.5 g of the crosslinking agent butanediol diglycidyl ether (BDDE) was added.

### [Comparative Example 3] Production of hyaluronic acid bead gels

A hyaluronic acid bead gel was produced in the same manner as in Comparative Example 1, except that 59.4 g of the crosslinking agent butanediol diglycidyl ether (BDDE) was added.

### [Comparative Example 4] Production of hyaluronic acid bead gels

Sodium hyaluronate (molecular weight: 1,000,000) at a concentration of 100 mg/ml was produced in 0.25 N 100 mL NaOH solution. This was dissolved overnight at 10 °C. 2 g of butanediol diglycidyl ether (BDDE), a crosslinking agent, was added. The reactant was dropped onto 900 g of olive oil phase. It was stirred at a speed of 700 rpm. It was reacted for 4 days at 25 °C. After 4 days, 500 ml of acetone was gradually added to collect the hyaluronic acid beads dispersed in the mixed solution. It was washed 3 times with 70% acetone. The collected hyaluronic acid beads were washed twice with 5L distilled water each.

The washed hyaluronic acid bead gels were swollen in PBS and sterilized at 121 °C to produce a hyaluronic acid bead gel.

### [Comparative Example 5] Production of hyaluronic acid bead gels

Sodium hyaluronate (molecular weight: 1,000,000) at a concentration of 100 mg/ml was added to 0.25 N 100 mL NaOH solution, and 0.5040 g of butanediol diglycidyl ether (BDDE) was added at the same time (Equivalent ratio of BDDE to hyaluronic acid repeating unit: 10 mol%). After mixing them for 30 minutes using a self-rotating centrifugal mixer, a first-step reaction was performed at 40 °C for 1 hour. The above reactant was dropped onto 200 g of isopropanol. While stirring the reactant at a speed of 700 rpm, a second-step reaction was performed at 40 °C for 24 hours.

After 24 hours, the dispersed hyaluronic acid beads were collected. Washing was performed 3 times with isopropanol. The collected hyaluronic acid beads were washed 5 times with 5L PBS each.

After pulverizing the washed hyaluronic acid bead gel, it was sterilized at 121 °C to produce a hyaluronic acid bead gel.

[Experimental Example] Comparison of physical properties of spherical hyaluronic acid bead gels produced in Examples 1, 2, and 3

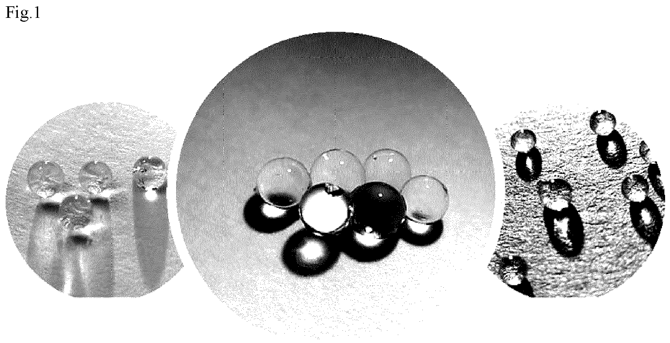

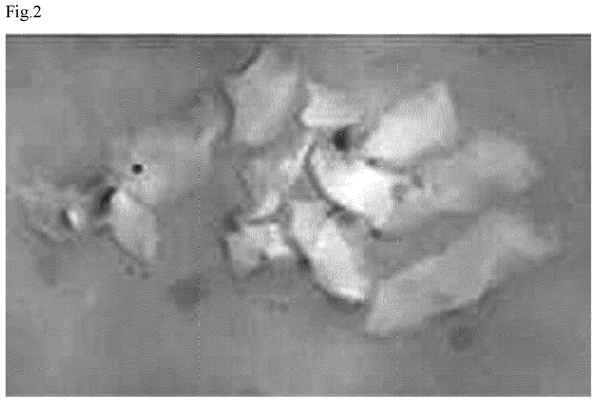

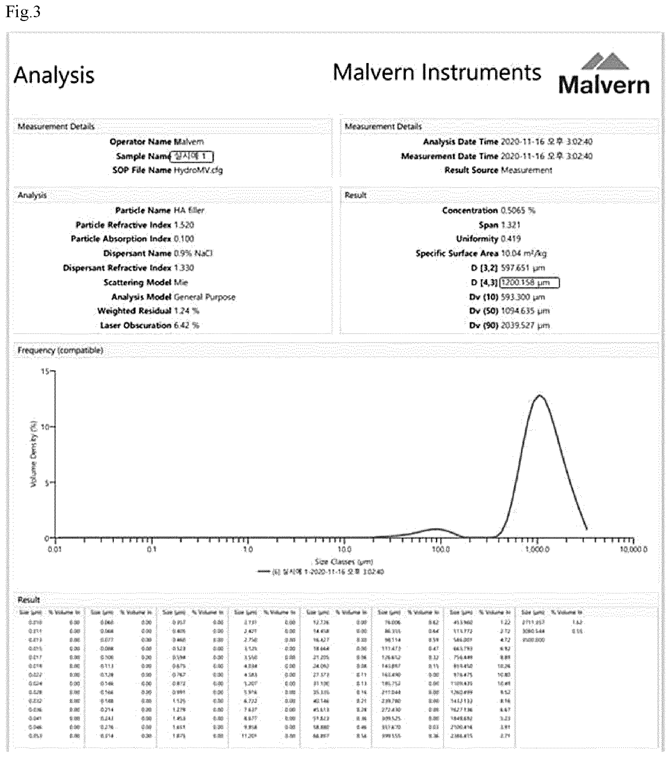

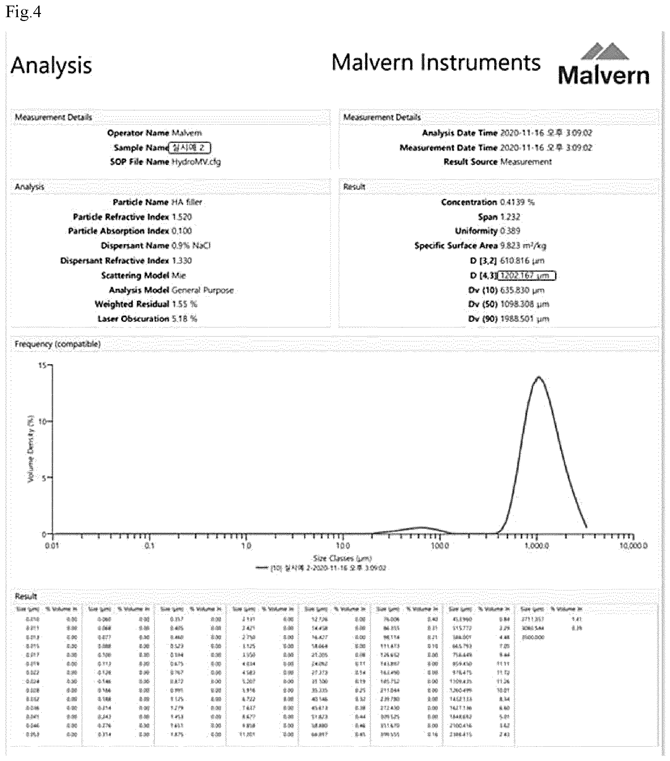

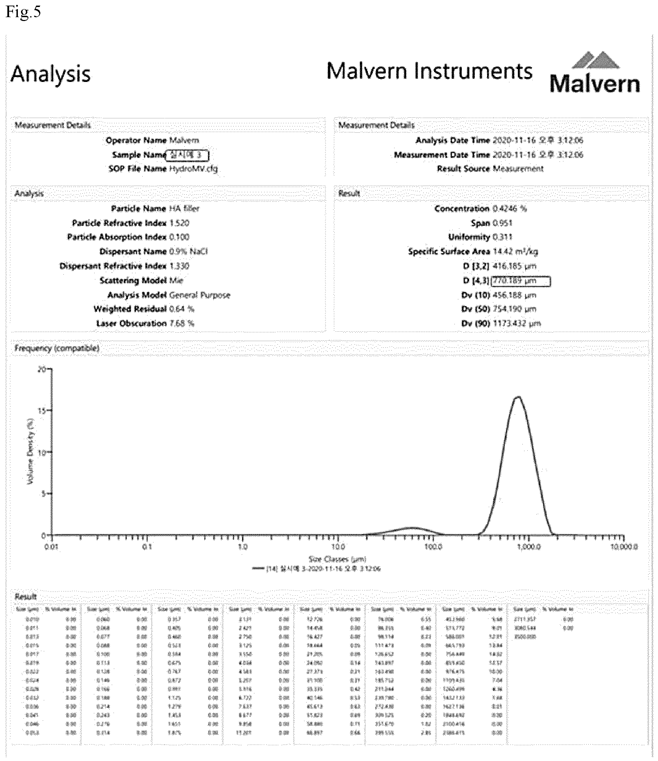

It was confirmed that the hyaluronic acid bead gels produced in Examples 1, 2, and 3 were spherical (FIG. 1), whereas the hyaluronic acid bead gel produced in Comparative Example 5 had an amorphous form in a generally produced formulation (FIG. 2). The particle size measured through particle size analysis was 1,200.158 µm in Example 1, 1,202.167 µm in Example 2, and 770.189 µm in Example 3, respectively (FIGS. 3, 4, 5).

The degrees of swelling of the spherical hyaluronic acid bead gel produced in Examples 1, 2, and 3 and Comparative Examples 2 and 3 were measured. The degree of swelling means the value obtained by dividing the weight of the hyaluronic acid bead gel swollen in PBS by the weight of the dry hyaluronic acid bead gel multiplied by 100. It was confirmed that the spherical shape was well maintained while having a high degree of swelling of 4,771 to 9,610%. The reason for such a high degree of swelling was that the crosslinking reaction was performed in two steps while using a small amount of the crosslinking agent so that the crosslinking reaction was performed with high efficiency and at the same time, it was possible to achieve through an advanced purification method of two or more, or three steps.

On the other hand, the beads produced in Comparative Examples 1 to 4 have too low degree of swelling of about 250 to 2,000 %, and thus show a disadvantage that safety problems may occur when applied to the human body. In addition, it was difficult to measure the degree of swelling of the beads produced in Comparative Example 5 due to the formation of an amorphous bead gel, and it was difficult to be used commercially, so the degree of swelling was not measured.

**[Table 1]**

| Degree of swelling of spherical hyaluronic acid bead gels | | | | | | | |
|---|---|---|---|---|---|---|---|
| | Example 1 | Example 2 | Example 3 | Comparative Example 1 | Comparative Example 2 | Comparative Example 3 | Comparative Example 4 |
| Degree of swelling(%) | 9,610 | 8,750 | 4,771 | 500 | 400 | 250 | 2,500 |

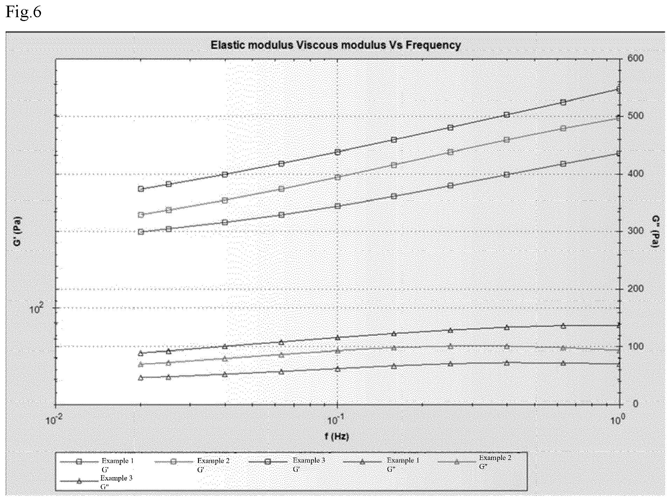

In order to confirm the rheological properties of the spherical hyaluronic acid bead gels produced in Examples 1, 2, and 3, viscoelasticity (complex modulus, storage modulus, loss modulus) at a frequency of 0.02 to 1 Hz using a rheometer was measured after sterilization, and as a result, it is expected to show suitable usability, safety, and performance as it shows similar figures to major products currently sold in the market (FIG. 6).

**[Table 2]**

| Comparison of rheological properties of spherical hyaluronic acid bead gels | | | |
|---|---|---|---|
| Rheology property (1Hz) | Example 1 | Example 2 | Example 3 |
| Complex modulus(G*, Pa) | 312.3 | 402.6 | 501.8 |
| Storage modulus(G', Pa) | 304.3 | 391.5 | 482.7 |
| Loss modulus(G", Pa) | 70.47 | 94.00 | 137.3 |

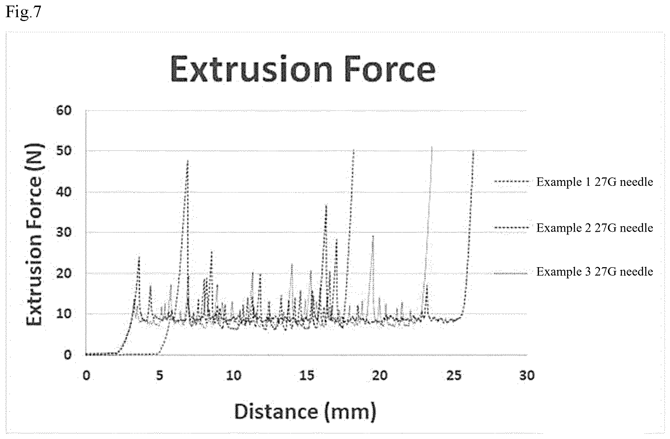

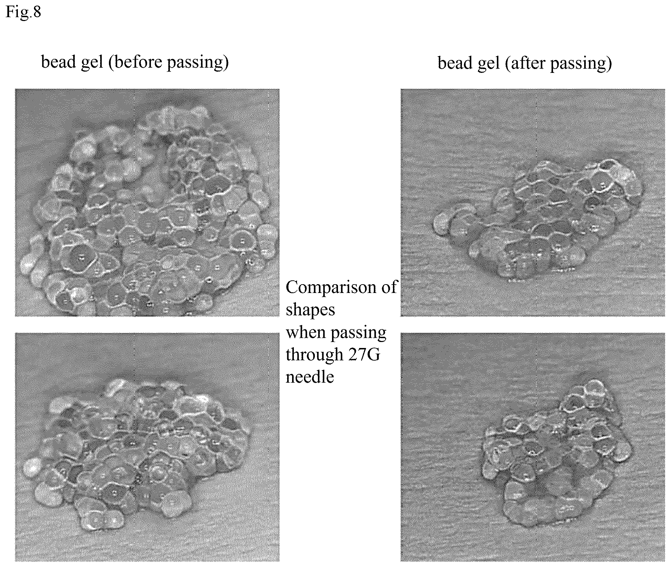

As a result of measuring the extrusion forces of the spherical hyaluronic acid bead gels produced in Examples 1, 2, and 3 (FIG. 7), in the case of Example 1, the extrusion force was 9.85 ± 3.64 N when a 27G needle was installed, in the case of Example 2, the extrusion force was 9.45 ± 2.79N when a 27G needle was installed, in the case of Example 3 the extrusion force was 9.69 ± 3.18N when a 27G needle was installed, which were similar to the extrusion force of 10 to 20 N of major products currently sold in the market. Referring to FIG. 8, it can be seen that the shape of the spherical hyaluronic acid bead gels produced in Examples 1, 2, and 3 did not change even after passing through the 27G needle and maintained the spherical shape well.

In addition, the hyaluronic acid bead gel that has undergone the swelling step is swollen again in an isotonic solution such as PBS, separated according to size, and produced singly or mixed and sterilized. Alternatively, the hyaluronic acid bead gels are collected in a precipitated state in an organic solvent or organic solvent aqueous solution, dried under nitrogen, air, heat, and reduced pressure, separated according to shape or size according to the purpose of application, and re-swelled in an isotonic solution such as PBS and sterilized.

The hyaluronic acid bead gel according to the present invention has various properties depending on the concentration of hyaluronic acid, the amount of crosslinking agent, alkali concentration, reaction temperature, reaction time, and the hyaluronic acid bead gel has a transparent and homogenous spherical shape, a high degree of swelling, high biocompatibility, is stable against heat and enzymes, and exhibits excellent viscoelasticity.

In addition, the present invention can stably obtain bead gels of various sizes by controlling the ratio of various oils and a hyaluronic acid solution without using emulsifiers, surfactants, solubilizers, and the like. In addition, in order to maintain a spherical shape in the obtaining step, a method of adding synthetic polymers such as PLGA and PCL, which are envelope forming materials, a method of producing an insoluble precipitate through ionic bonding, a method of precipitating in an organic solvent, or the like is not used. The spherical shape is maintained through two-step crosslinking in the hyaluronic acid solution dispersed in the oil phase without additives.

As described above, the hyaluronic acid bead gel according to the present invention is transparent, has a high degree of swelling, and exhibits uniform quality characteristics. Therefore, the spherical hyaluronic acid bead gel according to the present invention can be used as a filler for removing wrinkles, an implant, a drug delivery matrix, a cell carrier, a cell scaffold, an injection agent for j oint, cosmetics or the like.

## Claims

1. A method for producing a hyaluronic acid bead gel with a high degree of swelling, comprising:
(a) a reaction step of adding hyaluronic acid or a salt thereof in an alkali aqueous solution , adding a crosslinking agent, and then reacting a homogeneously mixed reactant;
(b) a crosslinking reaction step of forming a crosslinked covalent bond by stirring the reactant with oil; and
(c) a step of purifying a product obtained in the crosslinking reaction step,
wherein the hyaluronic acid bead gel is homogenously spherical.

2. The method of claim 1, wherein a degree (%) of swelling of the hyaluronic acid bead gel finally obtained through the purification process is 3,000 % or more.

3. The method of claim 1, wherein in the step (a), the crosslinking agent is added in a range of 1 to 30 equivalent % based on a repeating unit of the hyaluronic acid or salt thereof.

4. The method of claim 1, wherein in the step (a), a concentration of the hyaluronic acid or salt thereof in the reactant is 1 to 30 % by weight based on 0.1 to 10 N alkali aqueous solution .

5. The method of claim 1, wherein the step (a) is performed at a reaction temperature of 5 to 50 °C and a reaction time of 30 minutes to 24 hours.

6. The method of claim 1, wherein the step (a) is performed at a reaction temperature of 5 to 50 °C and a reaction time of 30 minutes to 24 hours.

7. The method of claim 1, wherein the step (b) is performed at a reaction temperature of 5 to 50 °C and a reaction time of 1 hour to 96 hours.

8. The method of claim 1, wherein in the step (b), a weight ratio of the hyaluronic acid or its salt alkali aqueous solution to which the crosslinking agent is added and the oil is 1:1 to 1:9.

9. The method of claim 1, wherein the crosslinking agent is one or more selected from the group consisting of 1,4-butandiol diglycidyl ether(BDDE), ethylene glycol diglycidyl ether(EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, and sorbitol polyglycidyl ether.

10. The method of claim 1, wherein the oil is one or more selected from the group consisting of mineral oil, medium chain triglycerides, menhaden oil, linseed oil, coconut oil, cod oil (fish oil), soya bean oil, whale oil, cotton seed oil, sesame seed oil, peanut oil, olive oil, lard oil, palms oil, corn oil, rapeseed oil, bone oil, china wood oil, and castor oil.

11. The method of claim 1, wherein the purification process includes (a) step of collecting the hyaluronic acid bead gel that has undergone the crosslinking reaction step; (b) a first purification step of washing the collected spherical gel with one or more selected from distilled water, an acidic solution, an aqueous solution containing an acid salt and PBS; (c) a second purification step of washing the hyaluronic acid bead gel that has undergone the first purification step with an organic solvent aqueous solution; (d) a third purification step of washing the hyaluronic acid bead gel that has undergone the second purification step with one or more selected from distilled water, an acidic solution, an aqueous solution containing an acid salt, and PBS; and (e) separating and collecting the hyaluronic acid bead gel obtained in the third purification step.

12. The method of claim 11, wherein the organic solvent is one or more selected from DMSO, DMF, acetonitrile, methanol, THF, acetone, an acetone aqueous solution and alcohol having 2 to 6 carbon atoms.

13. The method of claim 11, wherein the organic solvent aqueous solution used in the swelling step has a weight ratio of distilled water to an organic solvent of 9:1 to 1:9.
